# EUROPÄISCHE PATENTSCHRIFT

(11) **EP 3 993 659 B1**
(45) Veröffentlichungstag und Bekanntmachung des Hinweises auf die Patenterteilung: **22.11.2023**
(21) Anmeldenummer: 20737403.4
(22) Anmeldetag: 02.07.2020
(51) Int. Cl.: A41D 19/015, A61F 5/01, A41D 13/08

(54) **VORRICHTUNG IN ART EINER PERSÖNLICHEN SCHUTZAUSRÜSTUNG ZUR STABILISIERUNG UND ENTLASTUNG DES MENSCHLICHEN DAUMENSATTELGELENKES**
DEVICE IN THE FORM OF PERSONAL PROTECTIVE EQUIPMENT FOR STABILIZING AND RELIEVING THE HUMAN TRAPEZIOMETACARPAL JOINT
DISPOSITIF DE TYPE ÉQUIPEMENT DE PROTECTION INDIVIDUELLE POUR LA STABILISATION ET LA DÉCHARGE DE L'ARTICULATION TRAPÉZO-MÉTACARPIENNE HUMAINE

(30) Priorität: 04.07.2019 DE 102019209843
(43) Veröffentlichungstag der Anmeldung: 11.05.2022
(73) Patentinhaber: Fraunhofer-Gesellschaft zur Förderung der angewandten Forschung e.V., 80686 München (DE)
(72) Erfinder: LEFINT, Jérémy, 70569 Stuttgart (DE)
(74) Vertreter: Rösler, Uwe
(86) Internationale Anmeldenummer: PCT/EP2020/068641
(87) Internationale Veröffentlichungsnummer: WO 2021/001473

(56) Entgegenhaltungen:
- WO-A1-2014/148906
- DE-A1- 3 725 516
- DE-A1-102014 211 257

## Beschreibung

### Technisches Gebiet

Die Erfindung bezieht sich auf eine Vorrichtung in Art einer persönlichen Schutzausrüstung, kurz PSA, zur Stabilisierung und Entlastung des Daumensattelgelenkes einer menschlichen Hand. Die PSA weist eine ergonomisch an die Hand angepasste Halbschale auf, die einen proximalen, den Daumen-seitigen Bereich des Handgelenkes teilumfassenden Halbschalenabschnitt sowie einen den Mittelhandknochen des Daumens zumindest teilumfassenden distalen Halbschalenabschnitt vorsieht. Zu Zwecken der Fixierung der Halbschale an Daumen und Handgelenk ist jeweils ein mit dem distalen und proximalen Halbschalenabschnitt verbundenes Befestigungsmittel vorgesehen.

### Stand der Technik

Persönliche Schutzausrüstungen, kurz PSA, dienen der Vorbeugung von Verletzungs- oder Gesundheitsrisiken bei der Ausübung von Tätigkeiten. Derartige körpergetragene Hilfsmittel sollen im Unterschied zu klassischen medizinischen Orthesen den freien Bewegungsraum einer Person oder Körperteilen einer Person möglichst nicht einschränken.

Die weiteren Betrachtungen beziehen sich auf manuelle Tätigkeiten, bei denen die menschliche Hand und insbesondere der Daumen stark beansprucht werden, so dass ein entsprechender Schutz vor Verletzungen erforderlich ist. Manuelle Tätigkeiten mit kraftraubenden Greifvorgänge können in vielen Fällen zu Gelenkbeschwerden führen, die schleichend in Erscheinung treten und als solche nicht unmittelbar erkannt werden. Erst nach einer bestimmten Belastungsdauer können in der menschlichen Hand belastungsbedingte, biomechanische Gelenkirritationen, häufig in Form von akuten oder chronischen Entzündungen auftreten, die zu degenerativen Gelenkerkrankungen, bspw. in Form einer Daumensattelgelenkarthrose, führen können.

Bisher bekannte, diesbezügliche Schutzvorrichtungen sind als Orthesen ausgebildet, die vornehmlich als medizinisch therapeutische Hilfsmittel zur Unterstützung biomechanischer Gelenke dienen, deren primäre Funktion darin besteht, Gelenke zu stabilisieren, zu entlasten, zu führen oder zu immobilisieren. Aufgrund der Orthesen spezifischen starken Bewegungseinschränkung eignen sich derartige therapeutische Hilfsmittel nicht für den praktischen Einsatz zur Bewältigung üblicher oder alltäglicher Arbeiten.

Eine bekannte Mittelhand-Daumen-Orthese, die zur Ruhigstellung des menschlichen Daumens ausgebildet ist, ist in der Druckschrift DE 20 2014 007 828 U1 beschrieben. Die bekannte Orthese weist eine ergonomisch an dem Daumen und an das Handgelenk anlegbare starre Halbschale auf, deren dem Daumen und Handgelenk zugewandte Halbschalenoberfläche mit einer Polsterung belegt ist und deren proximaler und distaler Halbschalenendbereich jeweils mit einem Klettverschlussband an das Handgelenk bzw. im Bereich des ersten Daumenfingergliedes lösbarfest anbringbar ist.

Die Druckschrift EP 1 372 550 B1 offenbart eine Daumen-Handgelenk-Orthese, die eine handschuhartig ausgebildete Tragstruktur besitzt, mit einem zumindest den Daumen sowie die gesamte Handtellerfläche umgebenden Handschuhbereich, wobei zwischen Daumen und Handtellerbereich ein den Daumen in Richtung des Handtellers ziehendes Spannmittel eingearbeitet ist. Ferner erstreckt sich auf der dorsalen Daumenseite längs der Bahn des langen Daumenstreckmuskels eine mit der Tragstruktur verbundene Schiene aus elastischem Material, die so beschaffen ist, dass eine Abspreizbewegung des Daumens gegen die elastische Spannkraft in der Schiene auszuführen ist.

Die Druckschrift DE 36 31 253 A1 offenbart eine Handgelenkbandage zur Abstützung des Daumens, die einen den Daumen aufnehmenden, schlauchförmigen Daumenansatz aufweist. Am Daumenansatz der Bandage sind bis in den Bereich des Daumengrundgelenkes reichende Stützelemente handinnen- und außenseitig angeordnet. Die Stützelemente sind flexibel und schienenförmig ausgebildet.

Die Druckschrift WO 2016/011999 A1 offenbart eine Vorrichtung zur Linderung von Beschwerden von Daumensattelgelenkerkrankungen mit einer anatomisch angepassten Schiene aus Kunststoff, die einerseits am Handgelenk und andererseits unterhalb des ersten Daumengelenkes der erkrankten Hand lösbarfest fixierbar ist und im Bereich des dem Daumensattelgelenk zugewandt eine Pelotte vorsieht.

Die Druckschrift EP 1 813 233 B1 offenbart eine Daumen-Orthese, die als eine über den Daumen stülpbare, offen ausgebildete Daumenhülse vorsieht, längs der ein die Daumenbeweglichkeit unterstützendes Gelenkmittel eingebracht ist.

Der Druckschrift DE 20 2018 016 495 U1 ist eine Orthese zu entnehmen, die zwei starre und voneinander beabstandete, ergonomisch geformte Stabilisierungsabschnitte aufweist, die stoffschlüssig über einen flexiblen Beugungsabschnitt verbunden sind, der sich aus einer Vielzahl benachbart angeordneter Festkörpergelenke zusammensetzt.

Die Druckschrift DE 37 25 516 A1 offenbart einen Daumenschutz in Form eines entfernbaren Futterals zum Einsatz in die Daumentasche eines Handschuhs, um eine Verletzung des ellenseitigen Ligamentum collaterale des Daumens einer den Daumenschutz tragenden Person zu verhindern. Der Daumenschutz weist eine Rinne, die zwei über ein Scharnier schwenkbar gefügte Abschnitte enthält, sowie eine die Rinne umgebende Hülle auf, die eine Rückhaltevorrichtung enthält, wodurch eine Bewegung des Daumens in Richtung zur Speiche verhindert, jedoch in Richtung zur Elle erlaubt ist.

Die Druckschrift WO 2014/148906 A1 beschreibt eine Daumenschutzvorrichtung, die ein Handstützelement zur Aufnahme mindestens eines Handwurzelbereichs einer Hand, eine Daumenstützenstruktur, die eine Daumenoberkappe zur Aufnahme der Daumenspitze und ein flexibles Verbindungselement umfasst, das sich zwischen dem Handstützenelement und der Daumenkappe erstreckt, wobei das Verbindungselement ausreichend starr ist, um eine auf die Daumenkappe ausgeübte axiale Kraft auf das Handstützenelement zu übertragen.

Die Druckschrift DE 10 2014 211257 A1 offenbart u.a. eine Daumenorthese zur Führung und Begrenzung der Extensionsbewegung des Daumens mit einer ersten Aufnahme, zum Aufnehmen des ersten Daumenglieds sowie eine zweite Aufnahme, zum Aufnehmen des zweiten Daumenglieds, wobei beide Aufnahmen über ein Bindeglied beweglich miteinander verbunden sind. An jeder Aufnahme ist mindestens ein Anschlag derart vorgesehen, dass ein Anschlag einer Aufnahme mit einem Anschlag einer anderen Aufnahme in berührenden Kontakt bringbar ist, sofern eine maximal zulässige Bewegungsposition der Orthese erreicht ist.

### Darstellung der Erfindung

Der Erfindung liegt die Aufgabe zugrunde, eine Vorrichtung in Art einer PSA zur Stabilisierung und Entlastung des Daumensattelgelenkes einer menschlichen Hand mit einer ergonomisch an die Hand angepassten Halbschale, die einen proximalen, den Daumen-seitigen Bereich des Handgelenkes teilumfassenden Halbschalenabschnitt sowie einen den Mittelhandknochen des Daumens zumindest teilumfassenden distalen Halbschalenabschnitt aufweist, sowie mit jeweils einen mit dem distalen und proximalen Halbschalenabschnitt verbundenen Befestigungsmittel zur Fixierung der Halbschale am Daumen und Handgelenk, derart weiterzubilden, dass einerseits beim Tragen der PSA die Daumenbeweglichkeit nicht oder möglichst nicht eingeschränkt und zugleich ein effektiver Schutz des Daumens vor belastungsbedingten Gelenkverletzungen gewährleitstet werden sollen. Andererseits soll die neuartige PSA eine therapeutische Wirkung auf das Daumensattelgelenk entfalten, um Gelenkentzündungen wirksam entgegenzutreten.

Die Lösung der der Erfindung zugrunde liegenden Aufgabe ist im Anspruch 1 angegeben. Den Erfindungsgedanken in vorteilhafter Weise weiterbildende Merkmale sind Gegenstand der Unteransprüche sowie der weiteren Beschreibung insbesondere unter Bezugnahme auf die Ausführungsbeispiele zu entnehmen.

Lösungsgemäß zeichnet sich eine Vorrichtung in Art einer PSA zur Stabilisierung und Entlastung des Daumensattelgelenkes einer menschlichen Hand nach den Merkmalen des Oberbegriffes des Anspruches 1 dadurch aus, dass die proximale Halbschale formstabil ausgebildet und die distale Halbschale gegenüber der proximalen Halbschale um wenigstens eine Achse schwenkbar gelagert ist. Darüber hinaus besitzt die Halbschale wenigstens einen sich vom proximalen zum distalen Halbschalenabschnitt erstreckenden Längsschnitt, der im Bereich des Übergangs vom proximalen zum distalen Halbschalenabschnitt ein der Handseite zugewandtes Profil in Art eines Knicks aufweist. Zudem weist die distale Halbschale einen stegartig ausgebildeten, sich in Richtung des Längsschnittes erstreckenden Schalenabschnitt auf, mit einem einstückig mit dem proximalen Halbschalenabschnitt verbundenen proximalen Stegende, dessen Form und Größe unter Maßgabe der Ausbildung des Festkörpergelenkes gewählt sind.

Die lösungsgemäße Halbschale ist vorzugsweise einstückig aus einem an sich formstabilen Material, vorzugsweise einem leichtgewichtigen Hartkunststoff oder faserverstärkten Kunststoff gefertigt, und sieht zwischen dem proximalen und distalen Halbschalenabschnitt eine Flexibilität, vorzugsweise in Art eines Festkörpergelenkes vor, die bzw. das ein Schwenken des distalen Halbschalenabschnittes um die wenigstens eine Achse gegenüber des proximalen Halbschalenabschnittes ermöglicht. In einer bevorzugten Ausführungsform ist das Festkörpergelenk derart ausgebildet, dass der distale Halbschalenabschnitt um einen virtuellen Raumpunkt relativ zum proximalen Halbschalenabschnitt dreidimensional verschwenkbar gelagert ist.

Konstruktions- und/oder materialbedingt verfügt die Flexibilität über eine ihr eigene Steifigkeit, die ausgehend von einem kraftfreien Zustand der Halbschale, in dem die Halbschale einen Formgrundzustand einnimmt, mit zunehmendem Verformungsgrad der Flexion, d. h. mit zunehmendem Grad des Schwenkens des distalen Halbschalenabschnittes relativ zum proximalen Halbschalenabschnitt, zunimmt. Auf diese Weise kann die natürliche Daumenbeweglichkeit erhalten bleiben, jedoch werden mechanische Daumenbeanspruchungen dahingehend reduziert, indem die Bewegungsamplitude des Daumens bei Flexion und Extension ohne Einschränkung der räumlichen Beweglichkeit reduziert wird, zumal die durch die Halbschale auf den Daumen wirkende Kraft, ausgehend von dem vorstehend erläuterten Formgrundzustand mit zunehmendem Bewegungsausmaß des Daumens exponential ansteigt und der Daumenbewegung entgegenwirkt. Diese, der Flexibilität zuordenbare Steifigkeit schützt den Daumen in gleicher Weise vor unkontrollierten Daumenbewegungen, bspw. bedingt durch Hängenbleiben des Daumens an Hindernissen während einer Handbewegung.

Neben der vorstehend erläuterten Daumenschutzfunktion der lösungsgemäß ausgebildeten PSA vermag das im Übergangsbereich zwischen dem proximalen zum distalen Halbschalenabschnitt angeordnete und der Hand- bzw. Daumenseite zugewandte Profil einen auf das Daumensattelgelenk gerichteten, therapeutisch wirksamen Anpressdruck lokal zu generieren. Hierdurch werden das Daumensattelgelenk stabilisiert, mögliche unnatürliche, bspw. krankhaft bedingte oder unkontrollierte Relativbewegungen zwischen Daumenmittelhandknochen und dem zugehörigen Handwurzelknochen am Ort des Gelenkspaltes des Daumensattelgelenkes unterbunden und einer sich möglicherweise im Bereich des Daumensattelgelenkes ausgebildeten Arthrose entgegengewirkt. Die Anbringung des Profils in Art eines Knicks überlappt zumindest teilweise, vorzugsweise vollständig mit der in der Halbschale eingebrachten Flexibilität, d. h. das Profil in Art eines Knicks ist am Ort der Flexibilität im Übergangsbereich zwischen proximalen und distalen Halbschalenabschnitt angeordnet.

Ähnlich zum vorstehend erläuterten Steifigkeitsverhalten der Flexibilität ist das Profil in Art eines Knicks derart ausgebildet, so dass vermittels durch das Profils in Art eines Knicks bei größer werdender Daumenbewegung um die wenigstens eine Achse ein zunehmender Druck auf den Bereich des Daumensattelgelenkes ausgeübt wird. Die von der Daumenbewegung abhängige, sich dynamisch ändernde und vorzugsweise auf den Gelenkspalt des Daumensattelgelenkes lokal einwirkende Kraft vermag das Daumensattelgelenk sowohl mechanisch zu stabilisieren und zu entlasten, als auch innerhalb des Gelenkspalt biomechanische Gelenkprozesse zu stimulieren, so dass möglichen Gelenkentzündungen und -arthrosebildungen entgegengewirkt werden kann.

In einer bevorzugten Ausführungsform einer lösungsgemäß ausgebildeten Halbschale, die das vorstehend erläuterte therapeutisch wirksame Profil in Art eines Knicks aufweist, ist der der Handseite zugewandte distale Halbschalenabschnitt zumindest längs des Längsschnittes konkav gekrümmt und das der Handseite zugewandte Profil in Form eines Knickes zumindest längs des Längsschnittes konvex gekrümmt. Der der Handseite zugewandte proximale Halbschalenabschnitt, der sich längs des Längsschnittes unmittelbar an das Profil in Form eines Knickes anschließt, kann geradlinig oder gleichfalls leicht konkav gekrümmt ausgebildet sein. Auf eine konkrete Ausgestaltung der lösungsgemäß ausgebildeten Halbschale wird ausführlich auf das nachstehend illustrierte Ausführungsbeispiel Bezug genommen.

Zum Zwecke einer möglichst einfachen, ortsfesten und belastbaren Anbringung der lösungsgemäßen Vorrichtung am Daumenbereich einer Hand, ist die Halbschale mit einer Tragstruktur in Art eines Handschuhs verbunden, den eine Person in üblicher Weise anzulegen vermag. Die handschuhartige Tragstruktur kann als normal ausgebildeter Fingerhandschuh oder in Art eines die Finger jeweils halb umschließenden Fahrradhandschuhs ausgebildet sein. Selbstverständlich sind auch davon abweichende handschuhartig ausgebildete Tragstrukturvarianten denkbar.

Die Halbschale ist vorzugsweise mit der in Art eines Handschuhs ausgebildeten Tragstruktur zumindest im Bereich des Handrückens und im Bereich des Handballens über eine flexible Fügeverbindung, die vorzugsweise in Form einer nachgiebigen Naht ausgebildet ist, verbunden.

Die nachstehenden Figuren erläutern ein bevorzugtes Ausführungsbeispiel für die Realisierung der lösungsgemäßen Vorrichtung in Art einer PSA zur Stabilisierung und Entlastung des Daumensattelgelenkes einer menschlichen Hand.

### Kurze Beschreibung der Erfindung

Die Erfindung wird nachstehend ohne Beschränkung des allgemeinen Erfindungsgedankens anhand von Ausführungsbeispielen unter Bezugnahme auf die Zeichnungen exemplarisch beschrieben. Es zeigen:
- Fig. 1a - d: lösungsgemäß ausgebildete Halbschale, dargestellt aus unterschiedlichen Betrachtungswinkeln,
- Fig. 2: Längsschnittdarstellung der lösungsgemäß ausgebildeten Halbschale,
- Fig. 3: lösungsgemäß ausgebildete Halbschale, verbunden mit einem Handschuh sowie
- Fig. 4a, b: Detaildarstellungen zu Illustration der Nahtverbindung zwischen Halbschale und Handschuh.

### Wege zur Ausführung der Erfindung, gewerbliche Verwendbarkeit

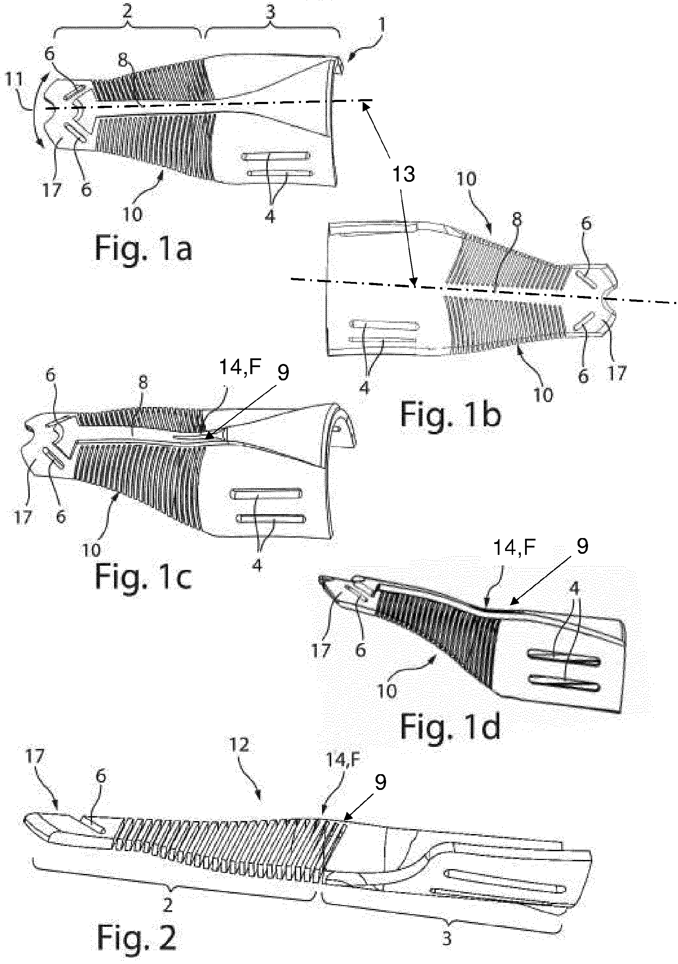

Die Figuren 1a bis d zeigen eine dreidimensionale Halbschale aus jeweils unterschiedlichen Betrachtungswinkeln. Figur 1a zeigt die Halbschale 1 von oben, Figur 1b von unten, Figur 1c schräg von oben und Figur 1d von der Seite. Die weiteren Erläuterungen beziehen sich gesamtheitlich auf die Bilddarstellungen in Figur 1a bis d.

Die Halbschale 1 ist unterteilbar in einen distalen Halbschalenabschnitt 2 sowie einen proximalen Halbschalenabschnitt 3, die beide einstückig miteinander verbunden sind.

Die Halbschale 1 ist aus einem formstabilen Material, vorzugsweise aus Kunststoff oder faserverstärktem Kunststoff, gefertigt. Zur Herstellung der Halbschale bieten sich an sich bekannte Spritzgussverfahren an, gleichwohl ermöglichen vor allem generative Herstellungsverfahren auf der Grundlage von ergonomischen Daumenformdaten Personen-spezifische Anpassungen der Halbschale.

Die der Hand zugewandte Innenseite 12, siehe Fig. 1b, der Halbschale 1 ist derart geformt, dass die Halbschale 1 im Bereich des proximalen Halbschalenabschnittes 3 in einem daumenseitig an die menschliche Hand angelegten Zustand das menschliche Handgelenk in palmarer sowie auch dorsaler Orientierung jeweils hälftig überdeckt. Der distale Halbschalenabschnitt 2 verfügt über eine Längserstreckung, die den Daumen seitlich zumindest im Bereich des Mittelhandknochens teilweise zu umfassen vermag.

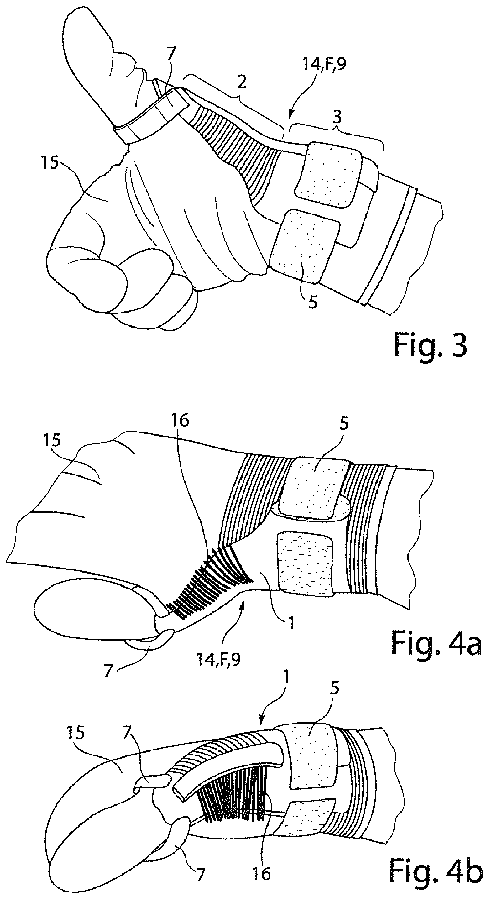

Die Halbschale 1 verfügt im Bereich des proximalen Halbschalenabschnittes 3 über schlitzförmige Öffnungen 4, durch die ein bandförmig ausgebildetes Befestigungsmittel 5, vorzugsweise in Form eines Klettbandes, siehe hierzu die Figuren 3, 4a,b, hindurchfädelbar ist.

In gleicher Weise sind am distalen Endbereich 17 des distalen Halbschalenabschnittes 2 entsprechende Öffnungen 6 für ein korrespondierendes Befestigungsmittel 7, siehe auch hier die Figuren 3, 4 a, b, eingebracht.

Im Gegensatz zu dem formstabil ausgebildeten, proximalen Halbschalenabschnitt 3 ist der distale Halbschalenabschnitt 2 aufgrund seiner strukturellen Ausbildung flexibel ausgebildet. Der distale Halbschalenabschnitt 2 weist einen im Wesentlichen stegartig ausgebildeten Schalenabschnitt 8 auf, dessen proximales Stegende 9 einstückig mit dem proximalen Halbschalenabschnitt 3 verbunden ist. Durch die Form- und Größenwahl des einstückig mit dem proximalen Halbschalenabschnitt 3 verbundenen proximalen Stegendes 9 ist eine lokale Flexibilität innerhalb der Halbschale geschaffen, durch die der distale Halbschalenabschnitt 2 gegenüber dem proximalen Halbschalenabschnitt 3 schwenkbar um wenigstens eine Achse, vorzugsweise um einen virtuellen Raumpunkt gelagert ist. Auf diese Weise behält der Daumen bei angelegter PSA seine natürliche Beweglichkeit, die durch die lösungsgemäße Halbschale 1 nicht oder nur unwesentlich eingeschränkt wird.

Der stegartig ausgebildete Schalenabschnitt 8 ist in seiner Längserstreckung aus Vollmaterial ausgebildet, d.h. im Längsschnitt 13, siehe Figuren 1a, b, weist der stegartig ausgebildete Schalenabschnitt keine Unterbrechungen oder Strukturierungen auf.

Beidseitig quer zur Längserstreckung des stegartig ausgebildeten Schalenabschnittes 8 erstrecken sich rippenartig ausgebildete Stegelemente 10, die jeweils einseitig einstückig mit dem stegartig ausgebildeten Schalenabschnittes 8 verbunden sind und sowohl einen mechanischen Schutz für den innenliegende Daumen bilden, als auch eine Daumenbeweglichkeit um den Bereich der Flexibilität F in der in Figur 1a angegebenen Lateralebene 11 ermöglichen.

Die dem Daumen zugewandte Schaleninnenseite 12 der Halbschale 1, die sowohl aus Figur 1b sowie auch aus Figur 2 hervorgeht, weist im Bereich der Flexibilität F ein der Handseite zugewandtes Profil in Art eines Knicks 14 auf, das Teil des stegartig ausgebildeten Schalenabschnittes 8 ist. Hierzu ist der der Handseite zugewandte distale Halbschalenabschnitt 2 zumindest längs des stegartigen Schalenabschnittes 8 konkav gekrümmt ausgebildet. Das der Handseite zugewandte Profil in Form eines Knicks 14 ist zumindest längs des Längsschnitts 13 lokal derart konvex gekrümmt, dass der stegartige Schalenabschnitt 8 an dieser Knickstelle 14 formschlüssig am Daumensattelgelenk, insbesondere im Bereich des Gelenkspaltes des Daumensattelgelenkes, zur Anlage kommt. Auf diese Weise wird der Gelenkspalt des Daumensattelgelenkes durch die mechanische Druckwirkung des Profil in Art eines Knicks 14 stabilisiert. Der proximale Halbschalenabschnitt 3 schließt sich an den Bereich der Flexibilität F bzw. des Profils in Art eines Knicks 14 vorzugsweise ohne signifikante Krümmung an.

Das vorstehend erläuterte Profil in Art eines Knicks 14 wird in unmittelbarer Anlage an den Bereich des Daumensattelgelenkes der menschlichen Hand gebracht und vermag bei Deformation der Halbschale 1 eine dynamisch, therapeutisch wirksame Kraft vorzugsweise auf den Gelenkspalt des Daumensattelgelenkes zu erzeugen. Je größer die Daumenflexion, d. h. die damit verbundene Auslenkung des flexibel gestalteten distalen Halbschalenabschnittes 2 um die Flexibilität F ist, umso größer wirkt der auf den Bereich des Daumensattelgelenkes gerichtete therapeutisch wirksame Druck.

Zur Anbringung der lösungsgemäß ausgebildeten Halbschale 1 an den Daumen einer menschlichen Hand eignet sich neben der Verwendung der jeweils bandartig bzw. klettbandartig ausgebildeten Befestigungsmittel 5, 7 eine Tragstruktur in Form eines Handschuhs, an die die Halbschale 1 gefügt ist.

Im Weiteren sei auf das in den Figuren 3, 4 a, b illustrierte Ausführungsbeispiel Bezug genommen, das eine als Handschuh ausgebildete Tragstruktur 15 vorsieht, an der die lösungsgemäß ausgebildete Halbschale 1 vermittels Befestigungsnähten 16, siehe Figur 4 a, b, befestigt ist. Zusätzlich ist aus den Figuren 3 und 4 die Darstellung der Befestigungsmittel 5, 7 zu entnehmen. So vermag gemäß der Figuren 3 , 4 das klettbandartig ausgebildete Befestigungsmittel 5 die Halbschale 1 am Unterarm bzw. Handgelenkbereich einer Person zu fixieren. Demgegenüber ermöglicht das am distalen Ende 17 des distalen Halbschalenabschnittes 2 angebrachte Befestigungsmittel 7 eine enge Fügung der Halbschale 1 längs des Daumens, so dass während sämtlicher Daumenbewegungen die Halbschale 1, insbesondere längs des distalen Halbschalenabschnittes 2, eng am Daumenrücken form- und kraftschlüssig anliegt. Beide Befestigungsmittel 5, 7 sowie die handschuhartig ausgebildete Tragstruktur 15 unterstützen den Halt der lösungsgemäß ausgebildeten Halbschale 1 an der Hand sowie unterstützen bei Flexion und Extension des Daumens die auf das Daumensattelgelenk der Person gerichtete therapeutische Druckwirkung. Die auf das Daumensattelgelenk wirkende Kraft verhält sich dabei exponentiell zum Bewegungsausmaß des Daumens. Insbesondere im Falle einer Daumenabspreizung, wie sie in Figur 3 illustriert ist, wird vermittels des Profil in Art eines Knicks 14 ein therapeutischer Druck auf das Daumensattelgelenk ausgeübt, der einer Arthrosenwirkung entgegenzuwirken vermag.

Neben den erläuterten Befestigungsmitteln 5, 7 ist die Halbschale 1 beidseitig zum stegartig ausgebildeten Schalenabschnitt 8 über Befestigungsnähte 16 möglichst flächig mit der als handschuhartig ausgebildeten Tragstruktur 15 verbunden. Die Befestigungsnähte 16 sind in Art einer Fischgrätstruktur ausgebildet, die eine Flexibilität aufweisen, so dass die Halbschale 1 im Bereich des distalen Halbschalenanschnittes 2 den Daumenbewegungen im natürlichen Bewegungsausmaß folgen kann. Die Befestigungsnähte 16 dienen neben ihrer strukturbedingten Flexibilität insbesondere der Fixierung des stegartig ausgebildeten Schalenabschnittes 8 längs des Daumenrückens, so dass aufgrund der ergonomischen Form und der Anbringung der Halbschale 1 relativ zum Daumen die frei Daumenbeweglichkeit gewährleistet werden kann.

Die Ausbildung der Tragstruktur 15 kann von der in den Figuren 3 und 4 illustrierten Fingerhandschuhform abweichen. Nicht notwendiger Weise müssen zur funktionsgerechten Anwendung der lösungsgemäßen Vorrichtung sämtliche Finger der Hand von der Tragstruktur 15 umfasst sein.

Die vorstehend erläuterte Halbschale kann als integraler Bestandteil einer individuell ausgebildeten persönlichen Schutzausrüstung verwendet werden, um bei Ausübung praktischer Tätigkeiten mechanischen Überlastungen im Daumenbereich zu dienen. Gleichsam ist es jedoch auch möglich, die lösungsgemäß ausgebildete Halbschale als reine medizinische Orthese auszubilden, die dem Schutz und der Rehabilitation eines arthrotischen Daumensattelgelenkes dient. Ferner ist es auch denkbar, die lösungsgemäße Halbschale in geeigneten handschuhartig ausgebildeten Tragstrukturen zu integrieren, wie sie bspw. bei vielen Sportarten, wie bspw. Skifahren, Radfahren o. ä. eingesetzt werden.

### Bezugszeichenliste

- 1: Halbschale
- 2: distaler Halbschalenabschnitt
- 3: proximaler Halbschalenabschnitt
- 4: Öffnungen
- 5: Befestigungsmittel
- 6: Öffnungen
- 7: Befestigungsmittel
- 8: stegartiger Schalenabschnitt
- 9: proximales Stegende
- 10: rippenartig ausgebildete Stegelemente
- 11: Lateralebene
- 12: Innenseite
- 13: Längsschnitt
- 14: Profil in Art eines Knicks
- 15: Tragstruktur
- 16: Befestigungsnaht
- 17: distaler Endbereich
- F: Flexibilität

## Patentansprüche

1. Vorrichtung in Art einer persönlichen Schutzausrüstung (PSA) zur Stabilisierung und Entlastung des Daumensattelgelenkes einer menschlichen Hand mit einer ergonomisch an die Hand angepassten Halbschale (1), die einen proximalen, den Daumen-seitigen Bereich des Handgelenkes teilumfassenden Halbschalenabschnitt (3) sowie einen den Mittelhandknochen des Daumens zumindest teilumfassenden distalen Halbschalenabschnitt (2) aufweist, sowie jeweils einem mit dem distalen und proximalen Halbschalenabschnitt (2, 3) verbundenen Befestigungsmittel (5) zur Fixierung der Halbschale (1) an Daumen und Handgelenk,
wobei die proximale Halbschale (3) formstabil ausgebildet und die distale Halbschale (2) gegenüber der proximalen Halbschale (3) um wenigstens eine Achse schwenkbar gelagert ist, und wobei die Halbschale (1) wenigstens einen sich vom proximalen zum distalen Halbschalenabschnitt (2, 3) erstreckenden Längsschnitt (13) besitzt, der im Bereich des Überganges vom proximalen zum distalen Halbschalenabschnitt (2, 3) ein der Handseite zugewandtes Profil in Art eines Knicks (14) aufweist,
**dadurch gekennzeichnet,**
**dass** die distale Halbschale (2) einen stegartig ausgebildeten, sich in Richtung des Längsschnittes (13) erstreckenden Schalenabschnitt (8) aufweist, mit einem einstückig mit dem proximalen Halbschalenabschnitt (3) verbundenen proximalen Stegende (9), dessen Form und Größe unter Maßgabe der Ausbildung des Festkörpergelenkes gewählt sind.

2. Vorrichtung nach Anspruch 1,
wobei zwischen dem proximalen und distalen Halbschalenabschnitt (2, 3) eine Flexibilität (F) vorgesehen ist, die ein Schwenken der distalen Halbschale (2) um die wenigstens eine Achse gegenüber der proximalen Halbschale (3) ermöglicht.

3. Vorrichtung nach Anspruch 2,
wobei der proximale und distale Halbschalenabschnitt (2, 3) einstückig verbunden sind und die Flexibilität (F) in Art eines Festkörpergelenkes ausgebildet ist.

4. Vorrichtung nach Anspruch 2 oder 3,
wobei die Flexibilität (F) mit dem Profil in Art eines Knicks (14) zumindest teilweise überlappt.

5. Vorrichtung nach einem der Ansprüche 2 bis 4,
wobei die Flexibilität (F) derart ausgebildet ist, dass ausgehend von einem kraftfreien Zustand der Halbschale (1), eine der Flexibilität (F) der Halbschale (1) zuordenbare Steifigkeit mit zunehmenden Grad des Schwenkens um die wenigstens eine Achse zunimmt.

6. Vorrichtung nach einem der Ansprüche 1 bis 5,
wobei das Profil in Art eines Knicks (14) derart gestaltet und angeordnet ist, dass vermittels des Profils in Art eines Knicks (14) bei größer werdender Daumenbewegung um die wenigstens eine Achse ein zunehmender Druck auf den Bereich des Daumensattelgelenkes wirkt.

7. Vorrichtung nach einem der Ansprüche 1 bis 5,
wobei der der Handseite zugewandte distale Halbschalenabschnitt (2) zumindest längs des Längsschnittes (13) konkav gekrümmt ist, und
dass das der Handseite zugewandte Profil in Form eines Knickes (14) zumindest längs des Längsschnittes (13) konvex gekrümmt ist.

8. Vorrichtung nach einem der Ansprüche 3 bis 7,
wobei das Festkörpergelenk am proximalen Halbschalenabschnitt (3) derart angeordnet und ausgebildet ist, dass der distale Halbschalenabschnitt (2) um einen virtuellen Punkt schwenkbar gelagert ist.

9. Vorrichtung nach einem der Ansprüche 1 bis 8,
wobei der proximale Halbschalenabschnitt (3) derart ausgebildet ist, dass der proximale Halbschalenabschnitt (3) in einem Daumen-seitig an die menschliche Hand angelegten Zustand das menschliche Handgelenk in palmarer und dorsaler Orientierung jeweils hälftig überdeckt.

10. Vorrichtung nach einem der Ansprüche 1 bis 9,
wobei beidseitig, sich quer zur Längserstreckung des stegartig ausgebildeten Schalenabschnittes (8) erstreckende und beidseitig längs des stegartig ausgebildeten Schalenabschnittes (8) mit diesem jeweils einseitig einstückig verbundene, rippenartig ausgebildete Stegelemente (10) angebracht sind.

11. Vorrichtung nach Anspruch einem der Ansprüche 1 bis 10,
wobei der stegartig ausgebildete Schalenabschnitt (8) ein distales Stegende aufweist, an dem mittel- oder unmittelbar das zur Fixierung der Halbschale (1) am Daumen dienende Befestigungsmittel (7) angebracht oder anbringbar ist.

12. Vorrichtung nach einem der Ansprüche 1 bis 11,
wobei der stegartig ausgebildete Schalenabschnitt (8) längs des Längsschnittes (13) unterbrechungsfrei aus Vollmaterial gefertigt ist.

13. Vorrichtung nach einem der Ansprüche 1 bis 12,
wobei das Profil in Art eines Knicks 14) in dem stegartig ausgebildeten Schalenabschnitt (8) im Bereich des proximalen Halbschalenabschnittes (3) eingebracht ist.

14. Vorrichtung nach einem der Ansprüche 1 bis 13,
wobei die Halbschale (1) mit einer Tragstruktur (15) in Art eines Handschuhs verbunden ist.

15. Vorrichtung nach Anspruch 14,
wobei die Halbschale (1) mit der in Art eines Handschuhs ausgebildeten Tragstruktur (15) zumindest im Bereich des Handrücken und im Bereich des Handballens über eine flexible Fügeverbindung verbunden ist.

## Claims

1. Device in the form of personal protective equipment (PPE) for stabilizing and relieving the trapeziometacarpal joint in a human hand, with a half shell (1) adapted ergonomically to the hand, having a proximal half shell section (3) which partially surrounds the thumb-side region of the wrist, and a distal half shell section (2) which at least partially surrounds the thumb metacarpal, and having a fastening means (5) connected to both the distal and proximal half shell sections (2, 3) for fixing the half shell (1) to the thumb and the wrist,
wherein the proximal half shell (3) is of dimensionally stable construction, and the distal half shell (2) is supported so as to be rotatable about at least one axis with respect to the proximal half shell (3),
and wherein the half shell (1) includes at least one lengthwise section (13) extending from the proximal to the distal half shell section (2, 3), which has a profile in the form of a deflection (14) closest to the hand side in the region of the transition from the proximal to the distal half shell section (2, 3),
**characterized in that** the distal half shell (2) has a shell section (8) of web-like design extending in the direction of the lengthwise section (13), with a proximal web end (9) connected integrally with the proximal half shell section (3), the shape and size of which are selected according to the structure of the flexure joint.

2. Device according to Claim 1, wherein a flexibility (F) is provided between the proximal and distal half shell sections (2, 3) that enables a rotation of the distal half shell (2) about the at least one axis with respect to the proximal half shell (3).

3. Device according to Claim 2, wherein the proximal and distal half shell sections (2, 3) are connected integrally and the flexibility (F) is designed in the manner of a flexure joint.

4. Device according to Claim 2 or 3, wherein the flexibility (F) at least partially overlaps with the profile in the manner of a deflection (14).

5. Device according to any one of Claims 2 to 4, wherein the flexibility (F) is designed in such manner that, starting from a force-free state of the half shell (1), a rigidity assignable to the flexibility (F) of the half shell (1) increases as the degree of rotation about the at least one axis increases.

6. Device according to any one of Claims 1 to 5, wherein the profile in the form of a deflection (14) is designed and arranged in such manner that, as the motion of the thumb about the at least one axis becomes greater, pressure on the region of the trapeziometacarpal joint increases under the effect of the profile in the form a deflection (14).

7. Device according to any one of Claims 1 to 5, wherein the distal half shell section (2) closest to the hand side is curved in concave manner at least along the lengthwise section (13), and
the profile in the form of a deflection (14) closest to the hand side is curved in convex manner at least along the lengthwise section (13).

8. Device according to any one of Claims 3 to 7, wherein the flexure joint on the proximal half shell section (3) is arranged and designed in such manner that the distal half shell section (2) is supported so as to be rotatable about a virtual point.

9. Device according to any one of Claims 1 to 8, wherein the proximal half shell section (3) is designed in such manner that, in a state in which the thumb-side is aligned against the human hand, the proximal half shell section (3) covers half the human wrist in both the palmar and the dorsal orientation.

10. Device according to any one of Claims 1 to 9, wherein web elements (10) of rib-like construction extending transversely to the longitudinal extension of the shell section (8) of web-like design, and each connected integrally to one side of the shell section (8) of web-like design along both sides are attached on both sides thereof.

11. Device according to any one of Claims 1 to 10, wherein the shell section (8) of web-like design has a distal web end, to which the fastening means (7) serving to secure the half shell (1) on the thumb is or can be attached indirectly or directly.

12. Device according to any one of Claims 1 to 11, wherein the shell section (8) of web-like design is made from solid material without interruption along the lengthwise section (13).

13. Device according to any one of Claims 1 to 12, wherein the profile in the form of a deflection (14) is incorporated in the shell section (8) of web-like design in the region of the proximal half shell section (3).

14. Device according to any one of Claims 1 to 13, wherein the half shell (1) is connected to a support structure (15) in the form of a glove.

15. Device according to Claim 14, wherein the half shell (1) is connected to the support structure (15) designed in the form of a glove via flexible joint connection at least in the region of the back of the hand and in the region of the heel of the hand.

## Revendications

1. Dispositif sous forme d'équipement de protection individuelle (EPI) pour stabiliser et soulager l'articulation du pouce d'une main humaine, comprenant une demi-coque (1) ergonomiquement adaptée à la main, qui présente une section de demi-coque proximale (3) englobant partiellement la zone côté pouce du poignet ainsi qu'une section de demi-coque distale (2) englobant au moins partiellement un os métacarpien du pouce, ainsi que des moyens de fixation (5) reliés aux sections de demi-coque distale et proximale (2, 3) pour fixer la demi-coque (1) sur le pouce et le poignet,
dans lequel la demi-coque proximale (3) est dimensionnellement stable et la demi-coque distale (2) est montée de manière pivotante par rapport à la demi-coque proximale (3) autour d'au moins un axe,
et dans lequel la demi-coque (1) possède au moins une section longitudinale (13) qui s'étend de la section de demi-coque proximale à distale (2, 3), qui présente dans la zone de transition de la section de demi-coque proximale à distale (2, 3) un profilé orienté vers le côté main à la manière d'un coude (14),
**caractérisé en ce que** la demi-coque distale (2) présente une section de coque en forme de bande (8) s'étendant dans la direction de la section longitudinale (13), avec une extrémité d'âme proximale (9) reliée d'un seul tenant à la section de demi-coque proximale (3), dont la forme et la taille sont choisies en fonction de la conception de l'articulation solide.

2. Dispositif selon la revendication 1, dans lequel entre la section de demi-coque proximale et distale (2, 3), une flexibilité (F) est prévue, qui permet à la demi-coque distale (2) de pivoter autour dudit au moins un axe par rapport à la demi-coque proximale (3).

3. Dispositif selon la revendication 2, dans lequel les sections de demi-coque proximale et distale (2, 3) sont reliées d'un seul tenant et la flexibilité (F) est conçue sous la forme d'une articulation solide.

4. Dispositif selon la revendication 2 ou 3, dans lequel la flexibilité (F) se superpose au moins partiellement au profilé à la manière d'un coude (14).

5. Dispositif selon une des revendications 2 à 4, dans lequel la flexibilité (F) est telle que, à partir d'un état sans effort de la demi-coque (1), une raideur attribuable à la flexibilité (F) de la demi-coque (1) augmente proportionnellement à l'augmentation du degré de pivotement autour dudit au moins un axe.

6. Dispositif selon une des revendications 1 à 5, dans lequel le profilé est conçu et disposé à la manière d'un coude (14) de telle sorte que, au moyen du profilé à la manière d'un coude (14), une pression croissante agisse sur la zone de l'articulation trapézo-métacarpienne à mesure que le mouvement du pouce augmente autour dudit au moins un axe.

7. Dispositif selon une des revendications 1 à 5, dans lequel la section de demi-coque (2) qui fait face au côté de la main est incurvée de manière concave au moins le long de la section longitudinale (13), et le profilé tourné vers le côté main est incurvé de manière convexe en forme de coude (14) au moins le long de la section longitudinale (13).

8. Dispositif selon une des revendications 3 à 7, dans lequel l'articulation solide sur la section de demi-coque (3) est disposée et conçue de telle sorte que la section de demi-coque distale (2) soit montée de manière pivotante autour d'un point virtuel.

9. Dispositif selon une des revendications 1 à 8, dans lequel la section de demi-coque proximale (3) est conçue de telle sorte que la section de demi-coque proximale (3) recouvre la moitié du poignet humain dans l'orientation palmaire et dorsale lorsqu'elle est placée du côté du pouce de la main humaine.

10. Dispositif selon une des revendications 1 à 9, dans lequel des éléments de bande configurés en forme de nervure (10) sont fixés, s'étendant des deux côtés, transversalement à l'extension longitudinale de la section de coque en forme de bande (8) et des deux côtés le long de la section de coque en forme de bande (8), en étant respectivement reliés en un seul tenant à celle-ci.

11. Dispositif selon une des revendications 1 à 10, dans lequel la section de coque en forme de bande (8) présente une extrémité distale de bande à laquelle est fixé ou peut être fixé directement ou indirectement le moyen de fixation (7) utilisé pour fixer la demi-coque (1) sur le pouce.

12. Dispositif selon une des revendications 1 à 11, dans lequel la section de coque en forme de bande (8) est fabriquée dans un matériau plein sans interruptions le long de la découpe longitudinale (13).

13. Dispositif selon une des revendications 1 à 12, dans lequel le profilé est réalisé à la manière d'un coude (14) dans la section de coque en forme de bande (8) au niveau de la section de demi-coque proximale (3).

14. Dispositif selon une des revendications 1 à 13, dans lequel la demi-coque (1) est reliée à la structure porteuse (15) à la manière d'un gant.

15. Dispositif selon la revendication 14, dans lequel la demi-coque (1) est reliée à la structure porteuse (15) à la manière d'un gant au moins au niveau du dos de la main et au niveau de la pointe de la main par l'intermédiaire d'une articulation flexible.
